# EUROPEAN PATENT APPLICATION

(11) **EP 3 520 802 A1**
(43) Date of publication of application: **07.08.2019**
(21) Application number: 18155229.0
(22) Date of filing: 06.02.2018
(51) Int. Cl.: A61K 36/9066

(54) **PROCESS FOR EXTRACTING CURCUMINOIDS**

(71) Applicant: EFESON - Chemical Research & Engineering Development, 41121 Modena (IT)
(72) Inventor: CASSINELLI, Erasmo, 41100 Modena (IT)
(74) Representative: Gregorj S.r.l.

(57) **Abstract**

The present invention relates to a process for extracting curcuminoids from turmeric, which comprises a step of clustering the curcuminoids with citric acid.

## Description

### Technical field of the invention

The present invention relates to a process for extracting curcuminoids from the turmeric plant.

### Prior art

It has been known for some time that turmeric naturally contains compounds, known as curcuminoids, which can be used as phyto-therapeutic principles due to their interesting biological properties.

Curcuminoids comprise curcumin, whose ketonic and enolic forms, respectively of formula (1a) and (1b), are in equilibrium 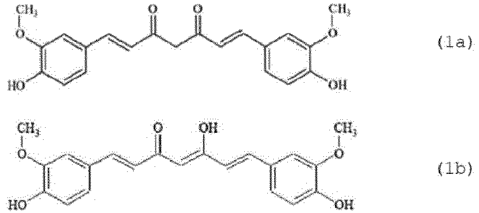 and demethoxycurcumin (DMC) and bis-demethoxycurcumin (BDMC), of formula (2) and (3), which are metabolites of curcumin, from which they differ by the absence of the methoxy group on one or on both the aromatic cycles, respectively.

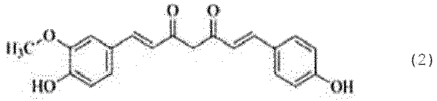

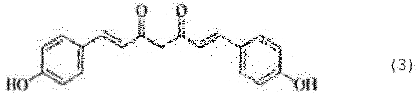

It must be noted that DMC and BDMC are represented here in their ketonic form, but they are also in equilibrium with the corresponding enolic form, in the same way as observed for curcumin.

Curcumin, also called curcumin I, is known for its many properties in medicine, including, for example, its antioxidant properties.

DMC, also known as curcumin II, is an excellent inhibitor of MCF-7 cells, responsible for breast cancer.

BDMC, also known as curcumin III, instead controls the MDR1 (multidrug resistance) gene expression, which is able to confer an MDR phenotype to tumour cells that have developed resistance to drugs. Gene expression is finely regulated by the cells. All steps of gene expression can be modulated, starting from step of transcription from DNA to RNA, up to post-translational modifications of the protein produced. Regulation of gene expression is fundamental for the cells, as it allows control of their internal and external functions. In practice, curcumin III increases the capacity to transport drugs to tumour cells and also exhibits important modifications in intestinal absorption or in the passage into the central nervous system.

It must be noted that although having very interesting properties from a biological view point, curcumin II and curcumin III are not currently commercially available.

One reason for this lack of availability of curcumin II and III lies in the known extraction processes, which require the use of alcohol, more precisely methanol (use of which has now been prohibited due to its toxicity), ethanol and ethylacetate. After extraction, the alcohol is evaporated and the curcuminoids obtained can be treated both to increase their concentration, and to make the active ingredients usable from a pharmacokinetic point of view.

Extractions with alcohol exhibit a certain selectivity towards curcuminoids. In fact, with this type of extraction, extracts are obtained that comprise a very high load of curcumin I, a minimum amount of curcumin II and almost no curcumin III.

Another problem with curcuminoids lies in their poor solubility in water, making them not readily bioavailable when included in drugs or food supplements to be taken orally.

Drugs taken orally pass through the gastrointestinal tract, to then enter the blood stream, passing through the cells that form the intestinal walls. The drugs are then transported in the blood to the liver, where they are metabolized. To support this long journey through hostile environments (owing to the presence of gastric juices in the stomach and digestive enzymes in the intestine), drugs must satisfy stringent requirements. One of these requirements is a correct water solubility and fat solubility equilibrium.

If the drug is too hydrophilic it will be unable to pass through the fat cell membranes of the intestinal wall. Instead, if it is too hydrophobic, i.e. too fatty, it will not be slightly soluble in the intestine and will dissolve in fat globules. This will result in a poor contact surface with the intestinal wall and consequently poor absorption into this wall.

The equilibrium between hydrophilicity and lipophilicity (solubility in fats) is quantified with the decimal logarithm of the partition coefficient P. A negative value for logP means the compound has a higher affinity for the aqueous phase, and inversely a positive value for logP denotes a higher concentration in the lipid phase, and therefore greater lipophilicity. If logP = 0, the compound is equally partitioned between the lipid and aqueous phases.

Curcumin I has a very positive logP, with a value equal to around 2.90.

Solutions to this problem have already been proposed. In particular, the use has been proposed of liposome systems that encapsulate the insoluble curcuminoids and create an emulsion that, through its ability to be present in water, is more easily transported into the tissues.

However, prior art solutions do not satisfy the requirements as liposome systems are only a vehicle for curcumin, but do not modify its properties, and in particular its hydrophobicity. Therefore, curcumin continues to be poorly available from a biological point of view.

### Brief summary of the invention

Therefore, the object of the present invention is to propose an extraction of curcuminoids that is not selective, does not require the use of solvents, has a low glycaemic index and a low glycaemic load, and that allows good retention of the substances extracted also in the membrane.

These and other objects are achieved by a process for extracting curcuminoids according to claim 1, by the composition according to claim 10 and by the compound according to claim 13.

The dependent claims define possible advantageous embodiments of the invention.

### Brief description of the drawings

For a better understanding of the invention and appreciation of its advantages some examples of nonlimiting embodiments thereof will be described below, with reference to the accompanying figures, wherein:
Fig. 1 is a chromatogram of a composition obtained by means of a first variant of the extraction process according to the invention; and
Fig. 2 is a chromatogram of a composition obtained by means of a second variant of the extraction process according to the invention.

### Description of embodiments of the invention

A process for extracting curcuminoids from turmeric according to the invention comprises a step of clustering of the curcuminoids with citric acid (of structure (4)).

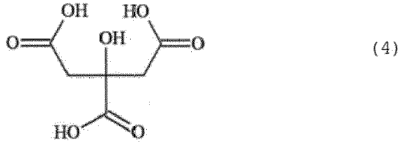

It is specified that, within the scope of the present invention, curcuminoids are meant as curcumin, demethoxycurcumin and bis-demethoxycurcumin. Therefore, the word curcuminoid refers to one of these three compounds.

According to another important aspect of the invention, the extraction process provides for the use of natural metabolites, more precisely plant metabolites, in place of the solvents normally used for extractions. In particular, the process according to the invention provides for the use of an extraction solution that comprises citric acid. According to an advantageous embodiment of the invention, the extraction solution also comprises trehalose. The extraction solution may also comprise sugars.

In general, the extraction process comprises a step of immersing a turmeric in an extraction solution comprising citric acid; and a step of clustering the curcuminoids with citric acid.

It is believed that clustering takes place through the formation of hydrogen bonds between two of the ketone functions of the citric acid and the enolic function of the curcuminoids located on their linear chain, in the their enolic form, as shown in formula 5 below.

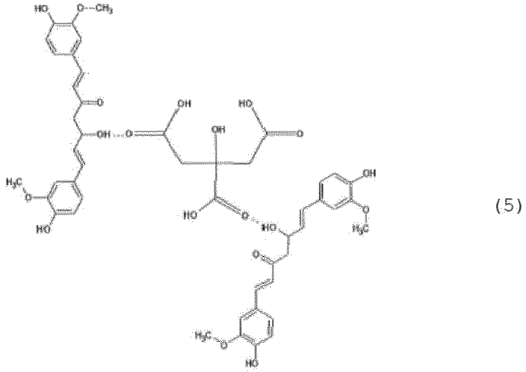

It can be seen from the structure of the cluster that it has a citric acid:curcuminoid molar ratio of 1:2.

The advantages of the extraction process according to the invention are many. The extracts obtained have a high total concentration in curcuminoids, and above all are not as selective as prior art extraction processes.

It has in fact been verified that the extracts obtained contain between 7 and 40 % by weight of curcuminoids, of which between 1.5 and 17 % by weight of curcumin I, between 1.5 and 10 % by weight of curcumin II, and between 4 and 13 % by weight of curcumin III.

Another advantage of the process according to the invention lies in the fact that the cluster of curcuminoid with citric acid obtained is soluble and stable in water. It has been measured that this cluster has a value of logP equal to around 0.95 in octanol. This value shows that the cluster obtained, besides being soluble in water, also has lipophilic character, which makes it available from a pharmacological point of view.

In accordance with a possible embodiment, the clustering step provides for exposing the turmeric to microwaves, the turmeric being at least partially immersed in the extraction solution.

According to an advantageous embodiment, the extraction solution comprises a mixture of trehalose and citric acid.

It has in fact been verified that with this mixture of trehalose and citric acid (eutectic mixture) the amount of curcuminoids extracted is higher with respect to the amount extracted with an extraction solution comprising only citric acid.

It is interesting to note that, in addition to the total amount of curcuminoids, partition of the curcuminoids extracted also changes. With an extraction solution comprising citric acid (without trehalose), curcumin I represents around 26%, curcumin II around 22% and curcumin III around 52% of the curcuminoids extracted. Instead, with the extraction solution comprising citric acid and trehalose, the percentages of curcumin I, II and III are around 43%, 24% and 33%, respectively. It can be seen that partition of the curcuminoids changes depending on the presence of trehalose, in particular in relation to curcumin I and curcumin III.

It can also be noted that with the extraction process according to the invention, curcumin II represents just under a quarter of the curcuminoids extracted, while curcumin III represents between one third and half of the curcuminoids extracted. This shows that the process is not selective in relation to the curcuminoids extracted, and also makes it possible to render curcumin II and curcumin III commercially available.

This increased extraction of curcumin III (increased with respect to known extraction processes) is due to the use of citric acid, which for its polarity interacts to a greater extent with curcumin III compared to the other two curcuminoids. In fact, this latter is more polar than curcumin II, which is in turn more polar than curcumin I.

According to a variant of the invention, in the case in which the extraction solution comprises citric acid and trehalose, it is provided that a step of exposing the extraction solution to microwaves takes place before the step of immersing the turmeric in the immersion solution. In this way, an amount of water is extracted from the eutectic mixture.

It is known that the amount of water present in eutectic mixtures influences their electrical conductivity, and therefore their polarity, as well as their viscosity. Some studies have shown that the maximum conductivity of a eutectic mixture is reached for an amount of water equal to around the 50 - 60 % by weight. The extraction of water allows the amount of water in the eutectic solution to be reduced so as to obtain a regression of conductivity, i.e. the eutectic mixture changes progressively from polar to nonpolar, thus exhibiting an improved extraction efficacy on all curcuminoids. By performing a first extraction of water before immersing the turmeric in the extraction solution, as mentioned above, the conductivity value of the eutectic mixture is modulated, so as to render it sufficiently polar to extract the most polar curcuminoids.

With regard to the step of exposing the turmeric to microwaves, to form the cluster of curcuminoids with citric acid, this preferably takes place at a power ranging between 500 and 1500 watts, more preferably between 700 and 1300 watts, even more preferably between 900 and 1100 watts.

This step of exposing the turmeric to microwaves preferably has a duration ranging between 10 and 180 minutes, more preferably between 20 and 120 minutes, even more preferably between 30 and 100 minutes. In general, the exposure time depends, among other things, on the power of the microwaves, a higher power requiring a lower exposure time, and vice versa.

The present invention also intends to protect the composition obtained by means of the extraction process as described above. This solution differs from the known mixtures of curcuminoids due to the proportions obtained between curcumin I, curcumin II and curcumin III, and in particular for the high percentages of curcumin II and curcumin III obtained.

This composition makes it possible to place on the market curcumin II and curcumin III, directly extracted from turmeric.

The present invention also intends to protect a composition comprising a mixture of curcumin, demethoxycurcumin and bis-demethoxycurcumin extracted from a turmeric plant.

Preferably, the composition comprises between 1.5 and 17 % by weight of curcumin, between 1.5 and 10 % by weight of demethoxycurcumin, and between 4 and 13 % by weight of bis-demethoxycurcumin.

Advantageously, the curcumin, demethoxycurcumin and bis-demethoxycurcumin are in the form of cluster with citric acid.

The present invention also intends to protect the cluster of curcuminoids with citric acid. More precisely, it intends to protect the cluster of curcumin, demethoxycurcumin or bis-demethoxycurcumin with citric acid.

This cluster is formed during the step of exposing the turmeric, immersed in the extraction solution containing citric acid, to microwaves. These microwaves provide the system with sufficient energy to promote cluster formation.

Identification of the formation of the clusters is carried out on a macroscopic basis of the extract obtained, in particular on the variation of the solubility of curcuminoids. It must be borne in mind that the logP of the extract obtained was measured at around 0.95 in octanol, while the curcuminoids usually have a logP equal to around 2.90.

It must also be noted that in the examples below the extracts were analysed using reverse phase uHPLC chromatography, that is using nonpolar column (c18) that allows identification of the different interactions of the curcuminoids with the nonpolar part supporting the need to change the polarity of the eutectic mixture.

It must also be borne in mind that clustering of the curcuminoids allows two hydrogen bond acceptor sites to be engaged, or "blocked". According to some known studies, this reduction of the number of hydrogen bond acceptor sites is in line with the increased pharmacological availability of the clusters of curcuminoids, linked to their improved solubilisation in water.

Preferably, the molar ratio between curcuminoid and citric acid is 2:1, as illustrated above.

The structure of the cluster shown above is suggested on the basis of chemical and mechanical calculations, which indicate how the ketone functions on the central chain are more susceptible to the formation of hydrogen bonds with respect to the groups present on the aromatic rings.

The present invention also intends to protect the cluster of curcumin, demethoxycurcumin or bis-demethoxycurcumin with citric acid for a use as drug or food supplement.

As stated above, the clusters obtained have a better equilibrium between hydrophilia and lipophilia with respect to the non-clustered curcuminoids, and as such are more suitable for a pharmacological use, whether this is in the form of drug or food supplement, in particular to be taken orally.

### Example 1

The curcuminoids are extracted from a fresh root of curcuma longa from Peru with an extraction solution comprising 90 g of citric acid and 200 g of water.

600 g of fresh root is added to the extraction solution. The mixture is then treated in a microwave oven at 900 watts for 80 minutes.

No water extraction has taken place. With this method 290 g of extract is obtained.

Considering that the fresh root comprises water for around half its weight, 7.48 g of curcuminoids were extracted from 300 g of dry turmeric, corresponding to 2.58 % by weight.

### Example 2

The curcuminoids are extracted from a fresh root of curcuma longa from Peru with an extraction solution comprising 500 g of citric acid, 500g of trehalose, and 400 g of water.

Due to the respective molar masses of citric acid (192.13 g/mol) and of trehalose (342.29 g/mol) a mixture of citric acid and trehalose in molar ratio of around 2:1 is obtained.

Before immersing the turmeric, the extraction solution is subjected to a first exposure to microwaves. Through this first exposure 300 g of water are extracted. As stated previously, this first extraction of water allows an optimized conductivity value of the eutectic mixture to be obtained, such as to render it sufficiently polar to allow improved extraction of the most polar curcuminoids, in particular of curcumin III, which is more polar than the other two curcuminoids.

Subsequently, 1 kg of fresh root of curcuma longa from Peru is added to the extraction solution. The ratio between extraction solution and root is therefore 1:1 by weight.

The mixture obtained is treated with microwaves at 1000 watts for 40 minutes. During this step a further 500 g of water is extracted from the turmeric root. This second extraction of water from the eutectic mixture determines a decrease of the conductivity of the eutectic mixture, and therefore of its polarity. By reducing the polarity of the eutectic mixture, this promotes extraction of curcumin II first, and then extraction of curcumin I. This change in the polarity of the eutectic mixture explains why the proportion of curcumin I extracted is greater with the eutectic mixture than with the citric acid alone, as will be seen below.

In this way 1 kg of extract is obtained, which is diluted in water in two steps, a first dilution in a ratio of 1:1, and a second in a ratio of 1:2. This dilution is implemented to proceed with analysis of the extract, to determine the amount of curcuminoids extracted.

With this extraction of 1 kg of fresh root (hence corresponding to around 500 g of dried turmeric) around 195 g of curcuminoids are obtained, corresponding to around 39 % by weight of the dried turmeric.

### Example 3

Quantitative analysis of the extracts obtained in examples 1 and 2 is carried out by means of UV-MS chromatography using a reverse phase column, in which the stationary phase comprises C18 and the eluent is a solution of acetonitrile and water. The chromatograms obtained with the extracts pursuant to examples 1 and 2 are illustrated in Figs. 1 and 2, respectively. The peaks a, b and c represent curcumin I, curcumin II and curcumin III, respectively. Chromatography was conducted at a wavelength equal to 425 nm.

After having calibrated the chromatograph in a manner conventional for the person skilled in the art, the amounts of curcuminoids extracted were calculated based on the surfaces of the areas of the peaks a, b and c.

It must be noted that the chromatograms obtained clearly show the different partitions of the curcuminoids according to the extraction solution used.

In the extraction of Example 1, curcumin I represents 26 % of the curcuminoids extracted, while in Example 2 the percentage rises to 43%. This difference is due to the second extraction of water, which takes place in the microwave treatment step in Example 2. By extracting water, the eutectic solution is made less polar and therefore extraction of curcumin I is improved with respect to curcumin II and curcumin III, due to their differences of polarity (it must be borne in mind that curcumin I is less polar than curcumin II, which in turn is less polar than curcumin III).

It must be stressed that the extraction process according to the invention is not banal as its development requires in-depth knowledge of the properties of eutectic mixtures, as well as an unconventional use of microwave radiations.

Table 1 below shows the contents of extracts of curcuminoids obtained according to known processes, and the results obtained in Examples 1 and 2 are shown as comparison.

**Table 1**

| Sample | Extraction solution | Average content of curcuminoids (% of the dry weight of turmeric) | | | |
|---|---|---|---|---|---|
| | | C | DMC | BDMC | Total |
| Rhizomes in India Kalimpong PTS-43 | Methanol | 2.19 | 1.58 | 1. 60 | 5.37 |
| | | 1.21 | 0.72 | 1.22 | 3.15 |
| Rhizome powder (commercial varieties available in India) Rajapurl Krishna Mydukur Salem Tekurpetta Armoor Ambehalada Ranahalada | ethanol | Mother/fin ger | | | |
| | | 4.31/4.77 | | | |
| | | 3.23/2.64 | | | |
| | | 5.71/4.18 | | | |
| | | 5.18/5.09 | | | |
| | | 4.94/4.58 | | | |
| | | 4.09/3.64 | | | |
| | | 0.09/0.06 | | | |
| | | 5.16/4.72 | | | |
| Rhizome powder (commercial varieties available in India) Salem Mysore Erode Balasore | Hexane/ methanol | 4.14 | 2.88 | 2.16 | 9.18 |
| | | 1.06 | 0.86 | 0.42 | 2.34 |
| | | 4.00 | 3.36 | 1.75 | 9.11 |
| | | 5.65 | 0.83 | 0.62 | 7.11 |
| Rhizomes in different areas in Thailand | Tetrahydrofura n/methanol | 1.28-6.02 | 0.92-3.80 | 1.10-5.18 | 3.30-14.99 |
| Rhizomes in Thailand | | 8.55-15.88 | 1.50-4.16 | 5.54-9.33 | 14.14-26.76 |
| Rhizomes in Thailand | methanol | | | | 0.46-10.23 |
| Rhizome powder: commercial varieties Madras Alleppey Jamaica | ethanol | 0.84 | 0.48 | 0.22 | 1.54 |
| | | 3.16 | 1.75 | 0.89 | 5.80 |
| | | 1.56 | 0.92 | 0.62 | 1.56 |
| Rhizome powder Ukon (Japan) Ukon (Guangdong, China) Turmeric ganter finger (Gunter, India) Jianghuang (Yunnan, China) Jianghuang (Yunnan, China) Kunir (Indonesia) Kunir (Indonesia) | methanol | 0.53 | 0.17 | 0.09 | 0.79 |
| | | 0.76 | 0.34 | 0.37 | 1.47 |
| | | 1.21 | 0.33 | 0.19 | 1.73 |
| | | 1.19 | 0.41 | 0.34 | 1.94 |
| | | 1.05 | 0.33 | 0.30 | 1. 68 |
| | | 1.36 | 0.48 | 0.54 | 2.38 |
| | | 1.38 | 0.43 | 0.39 | 2.20 |
| Rhizome powder: Korea | | Mother/fin ger | | | |
| | | 0.24/0.1 | | | |
| Rhizome powder: commercial products sold in Canada and the USA (various brands) | | 0.58-3.14 | | | |
| Extract: from commercial rhizome powder sold in Canada and the USA | Methanol | 21.4 | 7.2 | 5.1 | 33.7 |
| Commercial extracts | | 4.4-13.7 | 2.2-16.3 | 1.3-4.97 | 10.1-33.7 |
| Essential oil depleted turmeric fraction | oil Hexane/ methanol | 25.7 | 8.7 | 6.2 | |
| Commercial curcumin | | 74.2 | 14.9 | 4.5 | |
| Example 1 | Citric acid | 0.66 | 0.57 | 1.35 | 2.58 |
| Example 2 | Trehalose and citric acid | 16.92 | 9.54 | 12.78 | 39.24 |

In order to meet specific contingent requirements, the person skilled in the art can make numerous additions, modifications or replacements of elements with other functionally equivalent elements, to the described embodiments of the process for extracting curcuminoids according to the invention, without departing from the scope of the appended claims.

## Claims

1. A process for extracting curcuminoids from turmeric, comprising a step of clustering said curcuminoids with citric acid.

2. The process according to claim 1, comprising
a step of immersing a turmeric in an extraction solution comprising citric acid; and
a step of clustering the curcuminoids with citric acid.

3. The process according to claim 2, wherein the clustering step provides for exposing the turmeric, at least partially immersed in the extraction solution, to microwaves.

4. The process according to claim 2 or 3, wherein the extraction solution comprises a mixture of citric acid and trehalose.

5. The process according to claim 4, wherein the mixture of citric acid and trehalose is in a molar ratio of 1:2.

6. The process according to claim 4 or 5, comprising, before the step of immersing the turmeric in the extraction solution, a step of exposing said extraction solution to microwaves.

7. The process according to one or more of the claims 2 to 6, wherein the step of exposition to microwaves takes place at a power ranging between 500 and 1500 watts.

8. The process according to one or more of the claims 2 to 7, wherein the step of exposing said extraction solution to microwaves has a duration ranging between 10 and 180 minutes.

9. A composition obtained by means of the process according to one or more of the claims 1 to 8.

10. The composition comprising a mixture of curcumin, demethoxycurcumin and bis-demethoxycurcumin extracted from a turmeric plant.

11. The composition according to claim 10, comprising between 1.5 and 17 % by weight of curcumin, between 1.5 and 10 % by weight of demethoxycurcumin, and between 4 and 13 % by weight of bis-demethoxycurcumin.

12. The composition according to claim 10 or 11, wherein the curcumin, the demethoxycurcumin and bis-demethoxycurcumin are in the form of cluster with citric acid.

13. A cluster of curcumin, demethoxycurcumin of bis-demethoxycurcumin with citric acid.

14. The cluster according to claim 13, wherein the molar ratio between curcumin, demethoxycurcumin or bis-demethoxycurcumin and citric acid is 2:1.

15. The cluster of curcumin, demethoxycurcumin or bis-demethoxycurcumin with citric acid for use as drug or food supplement.
